# EUROPEAN PATENT APPLICATION

(11) **EP 3 026 414 A1**
(43) Date of publication of application: **01.06.2016**
(21) Application number: 14830127.8
(22) Date of filing: 14.07.2014
(51) Int. Cl.: G01M 13/04, A61B 6/03

(54) **TESTING DEVICE FOR THIN-WALLED LARGE BEARING**

(30) Priority: 23.07.2013 JP 2013152397
(71) Applicant: NTN Corporation, Osaka-shi, Osaka 550-0003 (JP); Kobayashi Machine Mfg. Co. Ltd., Yokkaichi-shi, Mie 510-0947 (JP)
(72) Inventor: KARASAWA, Miki, Kuwana-shi Mie 511-8678 (JP); KOHORI, Ken, Kuwana-shi Mie 511-8678 (JP); KAWASE, Yoshitsugu, Yokkaichi-shi Mie 510-0947 (JP)
(74) Representative: Maikowski & Ninnemann
(86) International application number: PCT/JP2014/068673
(87) International publication number: WO 2015/012139

(57) **Abstract**

A testing device (1) for a thin-walled large bearing includes: a face board (2) allowing the bearing (60) as a test target to be installed thereon; a face board support mechanism (3) supporting the face board (2) such that the face board (2) can be tilted around a tilting central shaft (12); an angle change drive device (4) for changing a tilt angle of the face board (2); and a bearing rotation motor (52) for rotating an inner ring of the bearing (60). The face board support mechanism (3) and the angle change drive device (4) are capable of changing the orientation of the face board (2) in a range from a horizontal orientation through a vertical orientation to a tilted orientation. The face board (2) is provided with a bearing installation mechanism (6) for installing the bearing (60) such that an outer ring of the bearing (60) is fixed.

## Description

### CROSS REFERENCE TO THE RELATED APPLICATION

This application is based on and claims Convention priority to Japanese patent application No. 2013-152397, filed July 23, 2013, the entire disclosure of which is herein incorporated by reference as a part of this application.

### BACKGROUND OF THE INVENTION

### (Field of the Invention)

The present invention relates to a testing device for conducting various tests of a thin-walled large bearing used in, for example, a CT scanner device which is a medical examination device.

### (Description of Related Art)

A CT scanner device (for example, Fig. 13 in Patent Document 1) has, as an examination unit, a circular rotating mount (gantry) which allows a cradle on which an examination subject such as a patient is laid to enter thereto and exit therefrom. The rotating mount is rotatably supported via a bearing on a fixing unit on the outer circumferential side, and is provided with an X-ray tube, an X-ray detector, and the like for capturing an image. The bearing used for supporting the rotating mount is a thin-walled large bearing with an outer diameter of about 600 to 1300 mm, and a load of about 1 t from mounted components such as the X-ray tube is applied to an inner ring of the bearing. Depending on a part to be imaged, such as an eyeball, the angle of the imaging needs to be changed, and therefore some CT scanner devices are configured such that the tilt angle of the rotating mount can be changed. In such CT scanner devices, the load applied to the bearing varies depending on the tilt angle of the rotating mount.

Generally, the bearing for supporting the rotating mount of the CT scanner device is required to have the following performances.
(1) Silence. This prevents an organ from being contracted due to patient's tension, and gives a sense of ease to the patient, to keep the organ acting normally.
(2) Low vibration. The lower the vibration is, the higher the quality of a captured image is.
(3) High speed. Shortening a time needed for imaging reduces the X-ray exposed dose.

In order to assure the above performances of the bearing for supporting the rotating mount, it is necessary to conduct a performance evaluation test before the bearing is incorporated into the CT scanner device. An example of such testing devices is disclosed in Patent Document 2. This testing device holds the bearing such that the bearing can be tilted at any angle, by a bearing holding mechanism, and allows an inner ring of the held bearing to be rotated at any rotation speed, thus enabling a performance evaluation test of the bearing under various conditions.

### [Related Document]

### [Patent Document]

[Patent Document 1] JP Laid-open Patent Publication No. 2012-82844
[Patent Document 2] JP Laid-open Patent Publication No. 2005-315681

Patent Document 2 describes that, since the baring can be tilted at any angle, the testing device is capable to make "the attachment orientation of the bearing be the same as that of, for example, a rolling bearing used for supporting a CT scanner gantry head". However, the specification of Patent Document 2 does not disclose that the bearing can be held horizontally. In addition, since the testing device is such a type that the bearing is fixed by being fitted into a depressed portion of a housing, the bearing can be fixed to the housing even when the bearing is in a vertical orientation. Thus, it is considered that, in this testing device, the bearing is attached to the bearing holding mechanism in a state in which the bearing is set in a vertical orientation or in a tilted orientation. Since the bearing used in the CT scanner device is large and heavy, it is not easy to attach the bearing to the bearing holding mechanism while a person supports the bearing in an appropriate orientation. In addition, when the bearing is in a vertical orientation or in a tilted orientation, a gap between the bearing holding mechanism and the bearing becomes uneven in the circumferential direction due to the influence of the weight of the bearing, and therefore it is difficult to attach the bearing at the center of the bearing holding mechanism.

### SUMMARY OF THE INVENTION

An object of the present invention is to provide a testing device for a thin-walled large bearing, that allows the thin-walled large bearing as a test target to be easily and accurately installed and allows a performance evaluation test to be conducted with the installed thin-walled large bearing tilted in any orientation.

A testing device for a thin-walled large bearing of the present invention includes: a face board configured to allow the thin-walled large bearing as a test target to be installed thereon; a face board support mechanism configured to support the face board such that the face board can be tilted around a tilting central shaft set to be horizontal, the tilting central shaft coinciding with or being parallel with a diameter line of the bearing installed on the face board; an angle change drive device configured to change a tilt angle of the face board; and a bearing rotation motor installed on the face board and configured to rotate an inner ring of the bearing. The face board support mechanism and the angle change drive device are capable of changing an orientation of the face board in a range from a horizontal orientation through a vertical orientation to a tilted orientation. The face board is provided with a bearing installation mechanism configured to install the bearing such that the bearing is placed on the face board and an outer ring of the bearing is fixed. The "thin-walled large bearing" as the test target in the present invention refers to a bearing in which a difference between an inner diameter and an outer diameter thereof with respect to the inner diameter is smaller than that of a general bearing, and the inner diameter is great, for example, a bearing in which a value of (outer diameter - inner diameter) / (inner diameter) is 0.3 or smaller and the inner diameter is 600 mm or greater.

According to the above configuration, the face board is set in the horizontal orientation by the face board support mechanism and the angle change drive device, and the bearing is installed by the bearing installation mechanism in a state in which the bearing is placed on the face board set in the horizontal orientation. Since the face board is horizontal, it is only necessary to lower the bearing set in the horizontal orientation from above onto the face board in installation of the bearing, and it is not necessary to support the bearing in a proper orientation by a person. Therefore, even if the bearing is the thin-walled large bearing, the bearing can be easily installed on the face board. In addition, when the bearing is placed on the face board set in the horizontal orientation, the weight of the bearing is uniformly applied to the face board. Therefore, position adjustment of the bearing along a bearing placement surface of the face board can be easily performed, and the bearing can be accurately installed at the center of the face board. Further, since the installation is performed in a state in which the bearing is placed on the face board, application to bearings having various outer diameters is easy, unlike a method in which a bearing is fitted into a face board.

After the bearing is installed on the face board, the face board is set in the vertical orientation or the tilted orientation, and the inner ring of the bearing installed on the face board is rotated by the bearing rotation motor, whereby a performance evaluation test of the bearing is conducted. Since the angle of the face board can be changed to any tilt angle or a predetermined tilt angle, the performance evaluation test of the bearing can be conducted in a state close to a usage state.

In the present invention, the bearing installation mechanism may include: a ring-shaped general-purpose frame which is detachably provided on the face board and is capable of selectively and concentrically fixing each of bearings having plural types of outer diameters; and a frame fixing module configured to fix the general-purpose frame to the face board. In this case, application to plural types of bearings having different outer diameters is enabled with a single bearing installation mechanism. In addition, the bearing can be fixed to the general-purpose frame at a place separated from the testing device, and the general-purpose frame to which the bearing is fixed can be fixed to the face board. Even if the type of the outer diameter of the bearing fixed to the general-purpose frame differs, operation for fixing the general-purpose frame to the face board is the same, and therefore work for installing the bearing on the face board is simplified.

The frame fixing module may include: a bolt insertion hole formed in the general-purpose frame or the face board; and a fixation bolt to be inserted into the bolt insertion hole so as to form an adjustment gap therebetween, to fix the general-purpose frame to the face board. A positioning mechanism may be provided which is configured to cause a center of the general-purpose frame to coincide with a center of the face board before fastening by the fixation bolt is performed. The positioning mechanism may be composed of: eccentric contact members rotatably provided at an eccentric position around a central axis perpendicular to the face board, to contact with respective plural positions of an outer circumferential surface of the general-purpose frame; and a rotation preventing module configured to switch the eccentric contact member between a rotation prevented state and a rotation allowed state.

In this case, the general-purpose frame is fixed to the face board through the following procedure. First, the general-purpose frame to which the bearing as the test target is fixed is placed roughly at a target installation position on the face board. At this stage, the general-purpose frame has not been fixed to the face board yet, and the general-purpose frame can be moved within a range of the adjustment gap between the bolt insertion hole and the fixation bolt. In this state, the center of the general-purpose frame is caused to coincide with the center of the face board by the positioning mechanism. Then, the fixation bolt is fastened to fix the general-purpose frame to the face board. The positioning mechanism is composed of plural sets of the eccentric contact member and the rotation preventing module, and is configured to cause the eccentric contact members to contact with the respective plural positions of an outer circumferential surface of the general-purpose frame, thereby positioning the general-purpose frame. Therefore, the mechanism is simple, and the positioning operation is easy.

In the present invention, a weight may be provided which is detachably attached to the inner ring of the bearing, to generate a moment load to the bearing. By providing the weight, the same moment load as that would be applied to the bearing in a usage state can be applied to the bearing.

In the present invention, a face board fixing mechanism configured to fix the face board to the face board support mechanism at any tilt angle or a predetermined tilt angle may be provided separately from the angle change drive device. By providing the face board fixing mechanism, the tilt angle of the face board can be prevented with high accuracy from being unintentionally changed during a test or the like.

Thus, the testing device for the thin-walled large bearing of the present invention is suitable for conducting a performance evaluation test of a bearing for supporting a rotating mount of a CT scanner device.

Any combination of at least two constructions, disclosed in the appended claims and/or the specification and/or the accompanying drawings should be construed as included within the scope of the present invention. In particular, any combination of two or more of the appended claims should be equally construed as included within the scope of the present invention.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a front view of a testing device for a thin-walled large bearing according to an embodiment of the present invention;
Fig. 2 is a side view of the testing device for the thin-walled large bearing;
Fig. 3A is a side view schematically showing a state in which a face board of the testing device for the thin-walled large bearing is in a vertical orientation;
Fig. 3B is a side view schematically showing a state in which the face board of the testing device for the thin-walled large bearing is in a tilted orientation;
Fig. 3C is a side view schematically showing a state in which the face board of the testing device for the thin-walled large bearing is in a horizontal orientation;
Fig. 4 is a side view of a face board fixing mechanism of the testing device for the thin-walled large bearing;
Fig. 5 is a view including a perspective view of the face board fixing mechanism and a partial enlarged view thereof;
Fig. 6 is a broken side view of a part of the testing device for the thin-walled large bearing;
Fig. 7A is a plan view of a positioning mechanism of the testing device for the thin-walled large bearing;
Fig. 7B is a side view of the positioning mechanism of the testing device for the thin-walled large bearing; and
Fig. 8 is a sectional view of an example of a CT scanner device.

### DESCRIPTION OF EMBODIMENTS

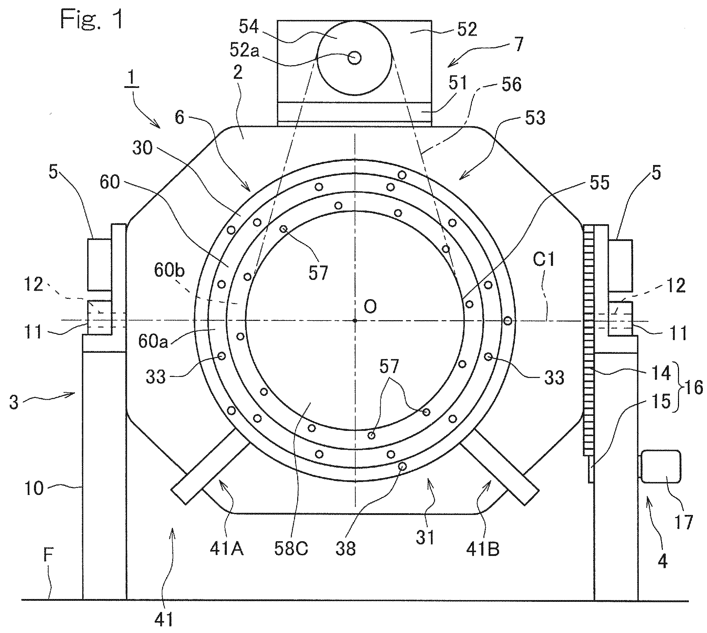

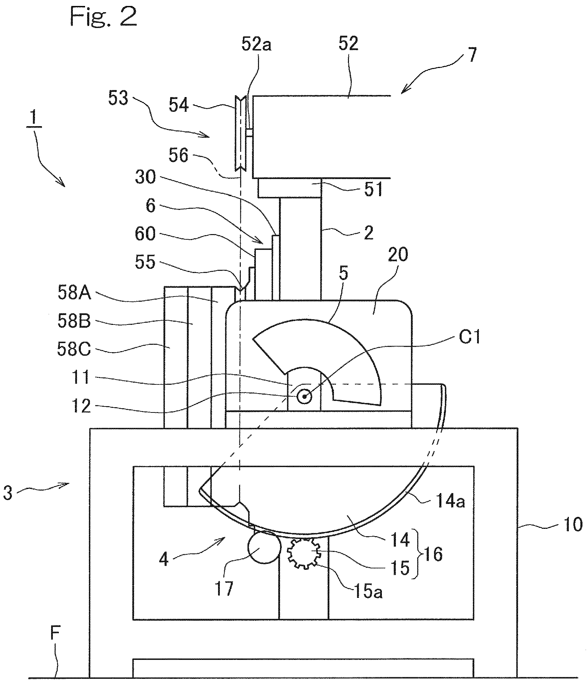

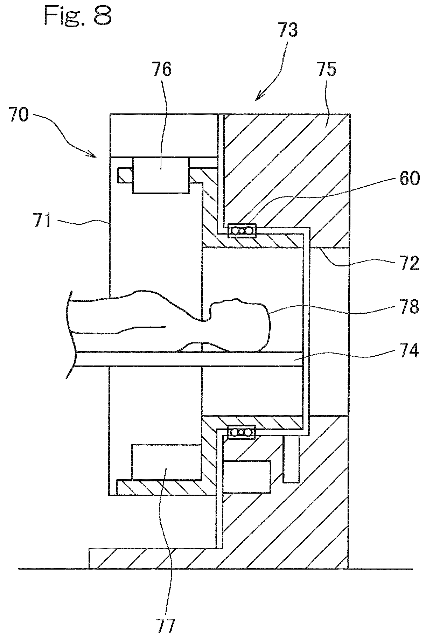

An embodiment of a testing device for a thin-walled large bearing according to the present invention will be described with reference to Fig. 1 to Fig. 7B. Fig. 1 is a front view of the testing device for the thin-walled large bearing, and Fig. 2 is a side view thereof. The testing device 1 for the thin-walled large bearing includes a face board 2, a face board support mechanism 3, an angle change drive device 4, a face board fixing mechanism 5, a bearing installation mechanism 6, and a bearing rotating mechanism 7. A bearing 60 as a test target to be subjected to a performance evaluation test by the testing device 1 is, for example, the thin-walled large bearing used for supporting a rotating mount 71 such as a gantry of a CT scanner device 70 shown in Fig. 8. The thin-walled large bearing is a rolling bearing such as a deep groove ball bearing, an angular contact ball bearing, a 4-point contact ball bearing, a cylindrical roller bearing, or a tapered roller bearing, for example. A specific configuration of the CT scanner device 70 will be described later.

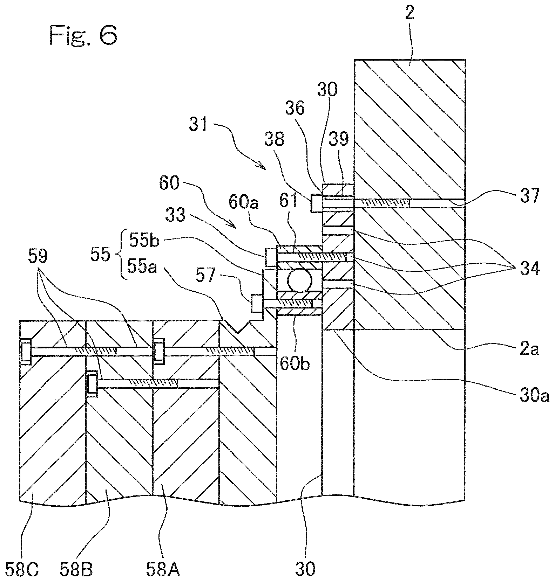

In Fig. 1 and Fig. 2, the face board 2 is used for installing thereon the bearing 60 as the test target, which is the thin-walled large bearing. In this example, the face board 2 is a ring-like plate body having an octagonal outer shape and having an opening 2a (Fig. 6) at the center thereof. The face board support mechanism 3 includes: a frame 10 placed on a floor surface F; and a pair of right and left tilting support bearing mechanisms 11 provided at an upper end of the frame 10, and rotatably supports a pair of tilting central shafts 12 protruding from right and left side surfaces of the face board 2, by the pair of right and left tilting support bearing mechanisms 11, respectively. A central axis C1 of the tilting central shaft 12 horizontally extends along the right-left direction, and coincides with a diameter line of the bearing 60 as the test target installed on the face board 2. Instead of coinciding with the diameter line of the bearing 60 as the test target, the central axis C1 of the tilting central shaft 12 may be parallel with the diameter line of the bearing 60 as the test target. By the face board support mechanism 3, the face board 2 is supported such that the face board 2 can be tilted around the central axis C1 of the tilting central shaft 12.

The angle change drive device 4 is a device for changing a tilt angle of the face board 2 around the central axis C1 of the tilting central shaft 12. The angle change drive device 4 includes a rotation transmission mechanism 16 composed of: a rotated element 14 fixed on one (in the example shown in the drawings, right one) of the right and left side surfaces of the face board 2; and a rotating element 15 provided on the frame 10. The angle change drive device 4 tilts the face board 2 together with the rotated element 14 by rotating the rotating element 15 by a rotational drive source 17 such as a motor provided on the frame 10. The rotation of the rotational drive source 17 is transmitted to the rotating element 15 via a reduction gear (not shown) such as a worm reduction gear so that the speed of the rotation is reduced. For example, the rotated element 14 is formed by attaching a chain 14a in a fixed manner to the outer circumference of a fan-shaped plate material centered on the central axis C1 of the tilting central shaft 12. The rotating element 15 is a sprocket having a claw 15a formed on the outer circumference thereof and to be engaged with the chain 14a. The rotated element 14 and the rotating element 15 may be gears having teeth engaged with each other.

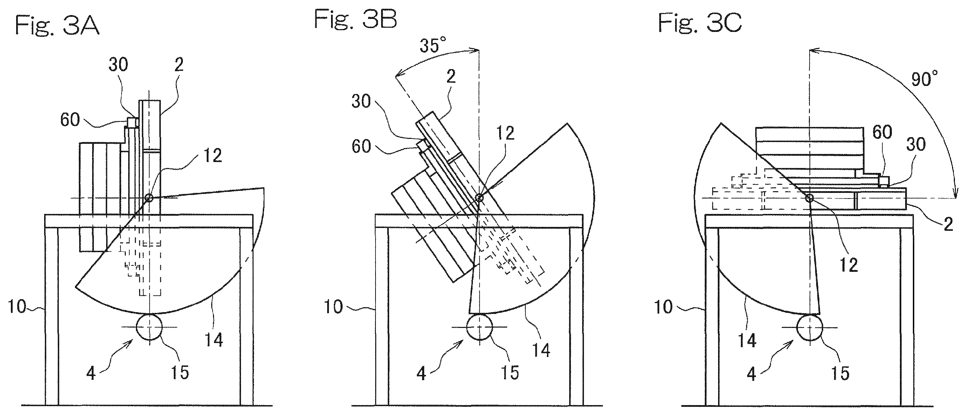

As shown in Fig. 3A to Fig. 3C, the orientation of the face board 2 can be changed in a range from a horizontal orientation through a vertical orientation to a tilted orientation by the angle change drive device 4. For example, as shown in Fig. 3A, using the vertical orientation as a reference, the orientation of the face board 2 can be changed from the tilted orientation (forward tilt at 35 degrees) shown in Fig. 3B to the horizontal orientation (backward tilt at 90 degrees) shown in Fig. 3C. By the way, the CT scanner device 70 (Fig. 8) conducts a test while changing the tilt of the rotating mount 71 (Fig. 8) in a range of about ± 30 degrees.

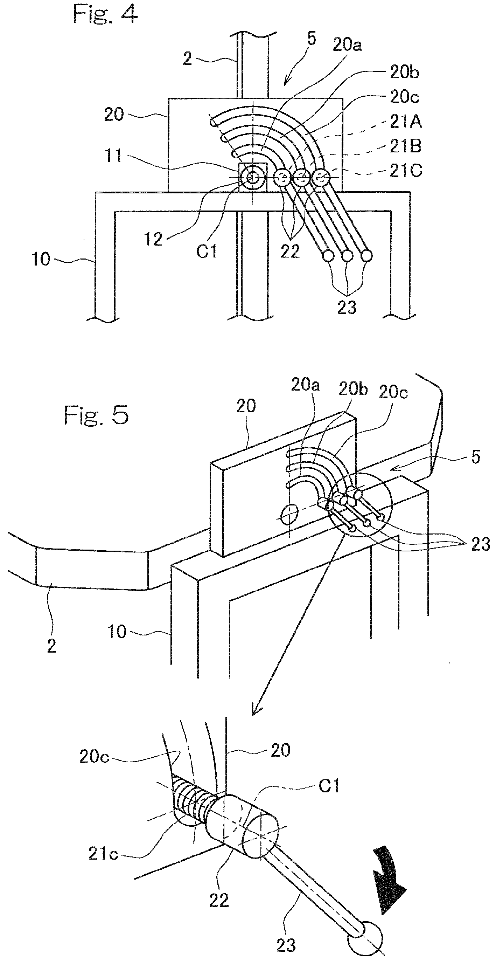

The face board fixing mechanism 5 is a mechanism for fixing the face board 2 to the face board support mechanism 3, at any tilt angle or a predetermined tilt angle. Fig. 4 and Fig. 5 show an example of the face board fixing mechanism 5. The face board fixing mechanism 5 shown in Fig. 4 has a fixation plate 20 provided on the frame 10 and having a plurality of arc-shaped guide grooves 20a, 20b, and 20c which are different in their radiuses and concentric about the central axis C1 of the tilting central shaft 12. Screw shafts 21A, 21B, and 21C with their ends screwed into a side surface of the face board 2 are inserted through the respective guide grooves 20a, 20b, and 20c of the fixation plate 20. Each screw shaft 21A, 21B, 21C is provided with a contact member 22 formed integrally therewith and having a greater diameter than those of the screw shafts 21A, 21B, 21C. A rotational operation lever 23 is attached to the contact member 22. The rotational operation lever 23 is turned to screw the screw shaft 21A (21B, 21C) into the face board 2 and cause the contact member 22 to strongly contact with the fixation plate 20, thereby fixing the face board 2 to the fixation plate 20 by means of a friction force between the contact member 22 and the fixation plate 20. Thus, the face board 2 is fixed at any tilt angle.

Instead of the above configuration, the screw shafts 21A, 21B, and 21C may be provided in a fixed state to the face board 2, and a nut (not shown) screwed to each screw shaft 21A, 21B, 21C may be fastened to fix the face board 2 to the fixation plate 20 by means of a friction force between the nut and the fixation plate 20. In the example in Fig. 4 and Fig. 5, three pairs of the guide grooves 20a, 20b, and 20c and the screw shafts 21A, 21B, and 21C are provided, but the number of the pairs is not limited to three. In the example in Fig. 4 and Fig. 5, the face board fixing mechanism 5 is provided at each of the right and the left, but may be provided at only one of the right and the left.

The bearing installation mechanism 6 is a mechanism for installing the bearing 60 as the test target in a state in which the bearing 60 is placed on the face board 2, and includes: a general-purpose frame 30 to which an outer ring 60a (Fig. 6) of the bearing 60 as the test target is fixed; and a frame fixing module 31 for fixing the general-purpose frame 30 to the face board 2. In the example in Fig. 6, the general-purpose frame 30 is a ring-shaped plate material having an opening 30a at the center thereof.

As shown in Fig. 6, fixation of the outer ring 60a to the general-purpose frame 30 is made by inserting a bolt 33 into a through hole 61 formed along the axial direction in the outer ring 60a, and then screwing a thread portion of the bolt 33 into a screw hole 34 formed in the general-purpose frame 30. A plurality of the through holes 61 and a plurality of the screw holes 34 are provided, along respective concentric circles, in the outer ring 60a and the general-purpose frame 30, respectively. In addition, in the general-purpose frame 30, a plurality of screw holes 34 are provided along each of a plurality of concentric circles having different diameters. Therefore, for one general-purpose frame 30, a bearing 60 having any outer diameter can be selected from among the bearings 60 as the test targets having plural types of outer diameters, and each of the bearings 60 can be concentrically installed on the one general-purpose frame 30 in an exchangeable manner, i.e., a selective manner. Through holes (not shown) may be formed along the axial direction in the face board 2 and the general-purpose frame 30, and a screw hole (not shown) may be formed in the outer ring 60a, whereby the outer ring 60a may be fastened to be fixed to the general-purpose frame 30 by a bolt (not shown) inserted from the face board 2 side. However, in light of handling performance, attachment performance, and the like, it is desirable that the outer ring 60a is fastened to be fixed to the general-purpose frame 30 by the bolt 33 inserted from the outer ring 60a side, as in the present embodiment.

The frame fixing module 31 is composed of: a bolt insertion hole 36 formed along the axial direction in the general-purpose frame 30; a screw hole 37 formed in the face board 2 so as to correspond to the bolt insertion hole 36; and a fixation bolt 38 to be inserted through the bolt insertion hole 36 and screwed into the screw hole 37. When the fixation bolt 38 is inserted into the bolt insertion hole 36, an adjustment gap 39 is formed therebetween at least in the radial direction. In order to form the adjustment gap 39, the bolt insertion hole 36 is formed to be a circular hole having a greater hole diameter than the diameter of the fixation bolt 38, or a long hole elongated in the radial direction.

The fixation bolt 38 inserted through the bolt insertion hole 36 is screwed into the screw hole 37 to fix the general-purpose frame 30 to the face board 2, and here, a positioning mechanism 41 (Fig. 1) is provided for causing the center of the general-purpose frame 30 to coincide with a center O of the face board 2 before the fixation bolt 38 is fastened. The positioning mechanism 41 is composed of two positioning mechanism portions 41A and 41B provided at two positions separated in the circumferential direction of the face board 2. The positions where the positioning mechanism portions 41A and 41B are provided are not particularly limited, but it is preferable that the positions thereof are lower than the tilting central shaft 12 when the face board 2 is tilted.

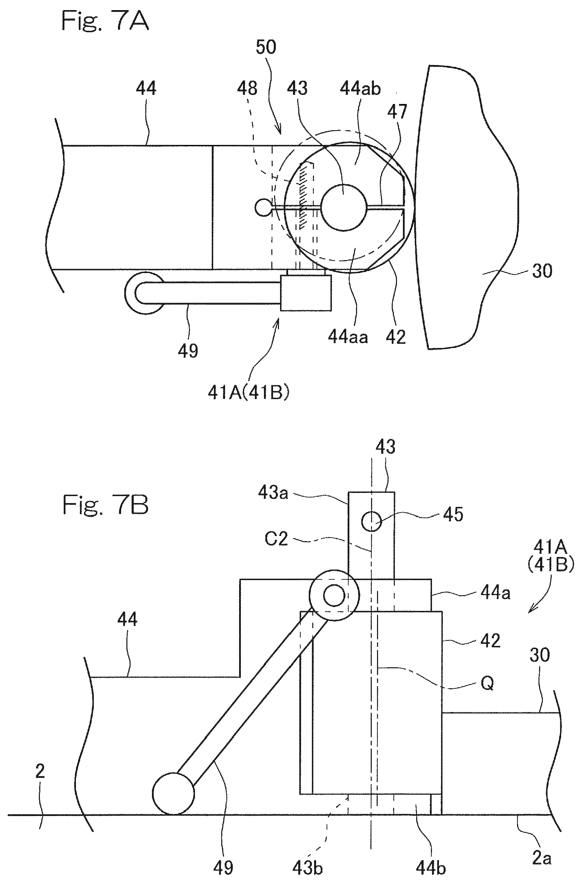

As shown in Fig. 7A and Fig. 7B, the positioning mechanism portions 41A and 41B each have an eccentric contact member 42 which is rotatable around a central axis C2 perpendicular to a general-purpose frame fixation surface 2a of the face board 2. Specifically, as shown in Fig. 7B, a rotation support shaft 43 having upper and lower portions 43a and 43b respectively extending upward and downward from upper and lower end surfaces of the eccentric contact member 42 is rotatably supported by a support member 44 fixed to the face board 2. The center of the rotation support shaft 43 coincides with the central axis C2. The eccentric contact member 42 has a cylindrical shape and the central axis C2 is eccentric with respect to a center Q of the cylinder. The upper portion 43a of the rotation support shaft 43 has an operation hole 45 penetrating in a direction perpendicular to the central axis C2. By inserting a tool (not shown) into the operation hole 45 and turning the tool, the eccentric contact member 42 can be rotated around the central axis C2.

As shown in Fig. 7A, an end side of an upper shaft support portion 44a of the support member 44 is divided via a slit 47 into two parts which become branched pieces 44aa and 44ab. A fastening screw 48 is inserted from one branched piece 44aa side, and a thread portion of the fastening screw 48 is screwed into the other branched piece 44ab. By turning a lever 49 attached to a base end of the fastening screw 48, the length by which the fastening screw 48 is screwed into the other branched piece 44ab is changed, whereby each of the branched pieces 44aa and 44ab is elastically deformed and the distance therebetween is changed. When the distance is shortened, as shown in Fig. 7B, the upper portion 43a of the rotation support shaft 43 is tightened, whereby rotation of the eccentric contact member 42 is prevented. When the distance is increased, the tightening of the upper portion 43a of the rotation support shaft 43 is released, whereby the eccentric contact member 42 is allowed to rotate. That is, the branched pieces 44aa and 44ab, the fastening screw 48, and the lever 49 shown in Fig. 7A compose a rotation preventing module 50 for switching the eccentric contact member 42 between a rotation prevented state and a rotation allowed state.

In Fig. 1 and Fig. 2, the bearing rotating mechanism 7 transmits rotation of a bearing rotation motor 52 provided on a bracket 51 fixed to the face board 2, to an inner ring 60b of the bearing 60 as the test target, via a belt transmission device 53. The belt transmission device 53 is composed of: a driving pulley 54 attached to an output shaft 52a of the bearing rotation motor 52; a driven pulley 55 fixed to the inner ring 60b of the bearing 60 as the test target; and a belt 56 wound around the driving pulley 54 and the driven pulley 55. The driven pulley 55 is provided concentrically with the inner ring 60b, and as shown in Fig. 6, is fixed to the inner ring 60b by a bolt 57 fastening a large-diameter portion 55b which protrudes toward the outer diameter side relative to a belt wound portion 55a. For example, the bearing rotating mechanism 7 is capable of rotating the inner ring 60b of the bearing 60 as the test target at a rotation speed of about 400 revolutions per minute.

As shown in Fig. 2, on a surface of the driven pulley 55 opposite to a surface thereof that contacts with the inner ring 60b of the bearing 60 as the test target, a plurality of weights 58A, 58B, and 58C for applying a moment load to the inner ring 60b are attached by bolts 59 in a stacked manner. The weight value of each of the weights 58A, 58B, and 58C and the number of the weights to be attached can be freely selected. Since the driven pulley 55 and the weights 58A, 58B, and 58C are located on the same side in the axial direction with respect to the bearing 60 as the test target, the moment load can be effectively applied to the bearing 60 as the test target. Sensors (not shown) for detecting respectively torque acting on the bearing 60 as the test target, and vibration, heat, sound, etc. generated during rotation are attached at corresponding appropriate positions in the testing device 1.

A usage method of the testing device 1 will be described. By the face board support mechanism 3 and the angle change drive device 4, the face board 2 is set in the horizontal orientation as shown in Fig. 3C. Then, the general-purpose frame 30 to which the bearing 60 as the test target has been fixed is installed on the face board 2 set in the horizontal orientation. The fixation of the bearing 60 as the test target to the general-purpose frame 30 may be performed at a place separated from the testing device 1.

In detail, the installation of the general-purpose frame 30 on the face board 2 is performed through the following procedure. First, the general-purpose frame 30 to which the bearing 60 as the test target has been fixed is placed roughly at a target installation position on the face board 2, using a crane or the like. Then, the fixation bolt 38 is inserted into the bolt insertion hole 36 of the general-purpose frame 30, and the fixation bolt 38 is screwed into the screw hole 37 of the face board 2. At this stage, the fixation bolt 38 is not completely screwed into the screw hole 37. Therefore, the general-purpose frame 30 has not been fixed to the face board 2 yet, and the general-purpose frame 30 can be moved within a range of the adjustment gap 39 between the bolt insertion hole 36 and the fixation bolt 38.

In this state, by the positioning mechanism 41, the general-purpose frame 30 is positioned such that the center of the general-purpose frame 30 coincides with the center O of the face board 2. The positioning method is as follows: the eccentric contact member 42 of each of the positioning mechanism portions 41A and 41B is set in a rotation allowed state, and is rotated so that the largest radius portion of the eccentric contact member 42 is directed toward the center O of the face board 2 as shown by a solid line in Fig. 7A. Thereafter, the eccentric contact member 42 is set in a rotation prevented state. Thus, in the case where the center of the general-purpose frame 30 placed at the rough position is positioned closer to the positioning mechanism portions 41A and 41B than the center O of the face board 2 is, each of a plurality of portions of an outer circumferential surface of the general-purpose frame 30 contacts with the largest radius portion of the eccentric contact member 42, whereby the center of the general-purpose frame 30 is positioned at a proper position so as to coincide with the center O of the face board 2. In the case where the center of the general-purpose frame 30 placed at the rough position is positioned farther from the positioning mechanism portions 41A and 41B than the center O of the face board 2 is, since there is a gap between the general-purpose frame 30 and the eccentric contact member 42, the general-purpose frame 30 is shifted on the face board 2 so as to fill the gap, whereby the general-purpose frame 30 is positioned at a proper position. After the positioning is finished, the fixation bolt 38 (Fig. 1) is fastened to fix the general-purpose frame 30 to the face board 2.

After the general-purpose frame 30 is fixed to the face board 2, the rotation prevented state of the eccentric contact member 42 is cancelled, and the eccentric contact member 42 is rotated to direct the smallest radius portion thereof toward the center O of the face board 2, so that the eccentric contact member 42 does not contact with the general-purpose frame 30. Thus, during a test, wearing of the bearing 60 as the test target due to fretting of the eccentric contact member 42 can be prevented.

Thus, in the case where the general-purpose frame 30 is installed on the face board 2 set in the horizontal orientation, it is only necessary to lower the general-purpose frame 30 from above onto the face board 2 in installation of the bearing, and it is not necessary to support the general-purpose frame 30 in a proper orientation by a person. Therefore, even if the bearing 60 as the test target is the thin-walled large bearing with a great weight, the general-purpose frame 30 can be easily installed on the face board 2. When the general-purpose frame 30 is placed on the face board 2 set in the horizontal orientation, a load from the bearing 60 as the test target and the general-purpose frame 30 is uniformly applied to the face board 2. Therefore, position adjustment of the general-purpose frame 30 along a bearing installation surface 2b of the face board 2 can be easily performed, and the bearing 60 as the test target can be accurately installed at the center of the face board 2.

After the general-purpose frame 30 is installed on the face board 2, the driven pulley 55 and the weights 58A, 58B, and 58C are attached to the inner ring 60b of the bearing 60 as the test target. The driven pulley 55 and the weights 58A, 58B, and 58C may be attached to the inner ring 60b of the bearing 60 as the test target before the general-purpose frame 30 is installed on the face board 2. Finally, the belt 56 is wound around the driving pulley 54 and the driven pulley 55, whereby test preparation is completed.

After the test preparation is completed, the face board 2 is set in the vertical orientation or the tilted orientation, to conduct a performance evaluation test of the bearing 60 as the test target. At this time, the face board 2 is fixed at a determined tilt angle by the face board fixing mechanism 5. Thereby, the tilt angle of the face board 2 can be reliably prevented from being unintentionally changed during a test or the like, thus ensuring security. The performance evaluation test is conducted by rotating the inner ring 60b of the bearing 60 as the test target by the bearing rotation motor 52 and then reading a value detected by each of the above sensors. Since the face board 2 can be changed at any tilt angle or a predetermined tile angle, the performance evaluation test of the bearing 60 as the test target can be conducted in a state close to the usage state.

The CT scanner device 70 shown in Fig. 8 will be described. The CT scanner device 70 includes: an examination unit 73 having an opening portion 72; and a cradle 74 movable in the opening portion 72. In the examination unit 73, the rotating mount 71 on the inner circumferential side is rotatably supported via two bearings 60 as rolling test targets on a fixation portion 75 on the outer circumferential side. The rotating mount 71 is provided with an X-ray tube 76 and an X-ray detector 77 located opposite to each other in the diameter direction. The cradle 74 on which an examination subject 78 such as a patient is laid is inserted into the opening portion 72 of the examination unit 73, the rotating mount 71 is rotated around the cradle 74 while X-rays are irradiated from the X-ray tube 76, and then the X-rays transmitted through the examination subject are detected by the X-ray detector 77, whereby a sectional image of the examination subject 78 is obtained.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the present invention as delivered from the claims annexed hereto, to be construed as included therein.

### [Reference Numerals]

1 ····testing device
2 ····face board
3 ····face board support mechanism
4 ····angle change drive device
5 ····face board fixing mechanism
6 ····bearing installation mechanism
7 ····bearing rotating mechanism
12 ····tilting central shaft
30 ····general-purpose frame
31 ····frame fixing module
41 ····positioning mechanism
42 ....eccentric contact member
50 ····rotation preventing module
52 ····bearing rotation motor
58A, 58B, 58C ····weight
60 ····bearing as test target
70 ····CT scanner device
71 ····rotating mount
C1 ····central axis of tilting central shaft
C2 ····central axis perpendicular to face board
O ····center of face board

## Claims

1. A testing device for a thin-walled large bearing, comprising:
a face board configured to allow the thin-walled large bearing as a test target to be installed thereon;
a face board support mechanism configured to support the face board such that the face board can be tilted around a tilting central shaft set to be horizontal, the tilting central shaft coinciding with or being parallel with a diameter line of the bearing installed on the face board;
an angle change drive device configured to change a tilt angle of the face board; and
a bearing rotation motor installed on the face board and configured to rotate an inner ring of the bearing, wherein
the face board support mechanism and the angle change drive device are capable of changing an orientation of the face board in a range from a horizontal orientation through a vertical orientation to a tilted orientation, and
the face board is provided with a bearing installation mechanism configured to install the bearing such that the bearing is placed on the face board and an outer ring of the bearing is fixed.

2. The testing device for the thin-walled large bearing, as claimed in claim 1, wherein
the bearing installation mechanism includes: a ring-shaped general-purpose frame which is detachably provided on the face board and is capable of selectively and concentrically fixing each of bearings having plural types of outer diameters; and a frame fixing module configured to fix the general-purpose frame to the face board.

3. The testing device for the thin-walled large bearing, as claimed in claim 2, wherein
the frame fixing module includes: a bolt insertion hole formed in the general-purpose frame or the face board; and a fixation bolt to be inserted into the bolt insertion hole so as to form an adjustment gap therebetween, to fix the general-purpose frame to the face board,
a positioning mechanism is provided which is configured to cause a center of the general-purpose frame to coincide with a center of the face board before fastening by the fixation bolt is performed, and
the positioning mechanism is composed of: a plurality of eccentric contact members rotatably provided at an eccentric position around a central axis perpendicular to the face board, to contact with respective plural positions of an outer circumferential surface of the general-purpose frame; and a rotation preventing module configured to switch the eccentric contact member between a rotation prevented state and a rotation allowed state.

4. The testing device for the thin-walled large bearing, as claimed in any one of claims 1 to 3, wherein
a weight is provided which is detachably attached to the inner ring of the bearing, to generate a moment load to the bearing.

5. The testing device for the thin-walled large bearing, as claimed in any one of claims 1 to 4, wherein
a face board fixing mechanism configured to fix the face board to the face board support mechanism at any tilt angle or a predetermined tilt angle is provided separately from the angle change drive device.

6. The testing device for the thin-walled large bearing, as claimed in any one of claims 1 to 5, wherein
the bearing is a bearing to support a rotating mount of a CT scanner device.
